# DEMANDE DE BREVET EUROPEEN

(11) **EP 4 159 186 A1**
(43) Date de publication de la demande: **05.04.2023**
(21) Numéro de dépôt: 22199445.2
(22) Date de dépôt: 03.10.2022
(51) Int. Cl.: A61K 8/02, A61K 8/44, A61Q 19/10, C11D 1/10

(54) **COMPOSITION COSMETIQUE SOLIDE**

(30) Priorité: 01.10.2021 FR 2110438
(71) Demandeur: Hyteck, 75006 Paris (FR)
(72) Inventeur: RUBERTI, Pascale, 13122 VENTABREN (FR)
(74) Mandataire: Tripoz, Inès

(57) **Abrégé**

L'invention concerne une composition solide destinée au nettoyage de la peau ou des phanères comprenant plus de 50% de tensioactifs, lesdits tensioactifs comprenant au moins trois acylglutamates sous forme de sels de sodium différents et ladite composition étant exempte de sels de triéthanolamine.

## Description

La présente invention se rapporte au domaine de l'hygiène et de la cosmétique, et plus précisément au domaine des compositions cosmétiques nettoyantes solides comprenant des tensio-actifs, utilisées pour nettoyer la peau ou les phanères.

Les compositions sous forme liquide comprennent de l'eau qui peut entrainer la possibilité de développements microbiens et des problèmes écologiques dus à l'utilisation de grandes quantités d'eau et à l'obligation d'utiliser des conservateurs.

Il y a également une demande croissante de compositions cosmétiques dont les ingrédients sont biosourcés et ne provoquent pas d'effets délétères sur la peau ou les phanères.

Il est donc important d'obtenir des compositions solides moussantes sous forme solide qui tout en préservant l'intégrité de la peau et des cheveux sont agréables à utiliser, c'est à dire qui se transforment rapidement en mousse et sont faciles à rincer.

Dans la cadre de la présente demande, un certain nombre de définitions doivent être données.

On entend par « solide » un objet caractérisé à l'échelle macroscopique par un volume et une forme déterminés, constants en l'absence de toute force extérieure ou condition extrême.

On entend par "pourcentage massique" une grandeur définie comme un pourcentage entre la masse d'un composé et la masse totale d'une composition le comprenant. Par exemple, si 10 grammes d'un composé y sont présents dans une composition z ayant une masse totale de 100 grammes, alors le pourcentage massique de y dans z est 10%.

On entend par "rapport massique" le rapport entre la masse de deux composés présents dans la même composition. Par exemple, si une composition comprend 3 grammes de composé f et 1 gramme de composé g, alors le rapport massique f/g est 3/1 soit 3.

Une composition selon l'invention peut comprendre également d'autres ingrédients, notamment des liants, des solvants, des agents absorbants, des agents de conditionnement de la peau, des agents de conditionnement capillaire, des stabilisateurs d'émulsion, des agents moussants, des émollients, des agents masquants, des régulateurs de pH, des opacifiants, ainsi que d'autres ingrédients cosmétiquement acceptables.

On connait de FR2894136 au nom de l'Oréal un article cosmétique soluble moussant comportant un support sous forme de fibres solubles dans l'eau et une composition solide comprenant au moins un tensioactif moussant choisi parmi les acylaminoacides et leurs dérivés. Cet article cosmétique présente notamment l'inconvénient de comprendre des fibres solubles servant de support qui sont notamment des fibres d'alcool polyvinylique et qui nuisent à la simplification de la composition et à l'élimination des constituants non essentiels pour obtenir des formulations respectueuses de l'environnement, car de récentes études ont montré l'impact néfaste de ces alcools sur les milieux aquatiques.

On connait de US6482782 au nom de Sung-O Kim des formulations solides qui comprennent également des tensioactifs choisis parmi les acylaminoacides et leurs dérivés, mais qui néanmoins comprennent des acides gras saponifiés ou savons comme le palmitate de sodium ou le laurate de sodium qui ne sont pas compatibles avec la fabrication de compositions sans savon ou « syndets ». De plus les acylglutamates sont présents sous forme de sels de triéthanolamine. La triéthanolamine ou plus communément appelée TEA est une amine tertiaire basique ; en l'utilisant comme contre-ion des acylglutamates, elle est utilisée comme ajusteur de pH, tout comme pour ses propriétés tensioactives et émulsifiantes, mais n'est pas respectueuse de l'environnement. Sa fabrication est extrêmement polluante, et de plus, elle est suspectée d'être toxique pour la peau. Lesdites compositions comprennent également un support pulvérulent, composé d'oxyde de titane, de fer ou encore de chrome, également non compatible avec des formulations respectueuses de l'environnement.

Il existe donc un besoin de composition cosmétique solide sans savon et sans support et qui soit satisfaisant quant à ses propriétés sensorielles (notamment le départ et l'onctuosité de la mousse) qui respecte la peau et les phanères tout en étant respectueuse de l'environnement.

Dans un mode de réalisation, la composition cosmétique solide selon l'invention est caractérisée en ce qu'elle comprend moins de 10% d'eau, en pourcentage massique par rapport à la masse totale de ladite composition cosmétique solide.

Dans un mode de réalisation, la composition cosmétique solide selon l'invention est caractérisée en ce qu'elle comprend moins de 5% d'eau, en pourcentage massique par rapport à la masse totale de ladite composition cosmétique solide. Dans un mode de réalisation, la composition cosmétique solide selon l'invention est caractérisée en ce qu'elle comprend moins de 2% d'eau, en pourcentage massique par rapport à la masse totale de ladite composition cosmétique solide. Dans un mode de réalisation, la composition cosmétique solide selon l'invention est caractérisée en ce qu'elle comprend moins de 1% d'eau, en pourcentage massique par rapport à la masse totale de ladite composition cosmétique solide.

Dans un mode de réalisation, les tensio-actifs sont choisis dans le groupe des tensio-actifs anioniques et plus particulièrement des acylglutamates sous forme de sels de sodium pour leurs propriétés non irritantes et non délipidantes. En effet, les acylglutamates, quelle que soit la chaîne grasse, n'éliminent pas les lipides du ciment intercellulaire essentiel au bon maintien en eau de la couche cornée en raison de leur pouvoir sélectif nettoyant.

Dans un mode de réalisation, la composition cosmétique solide selon l'invention est caractérisée en ce qu'elle est exempte de sels de triéthanolamine.

Dans un mode de réalisation, la composition cosmétique solide selon l'invention est caractérisée en ce qu'elle est exempte de savons ou de sels de sodium d'acide gras résultant d'une saponification comme le palmitate de sodium ou le laurate de sodium.

Dans la plupart des compositions cosmétiques solides destinées au nettoyage de la peau ou des phanères le tensioactif sodium cocoyl isethionate ou SCI qui est un tensioactif dérivé de l'huile de coco et présenté sous forme de poudre granulée est utilisé. Il s'utilise aussi en association avec le Sodium coco sulfate ou SLSA notamment pour formuler des shampooings solides tout en apportant une mousse riche et crémeuse. Bien qu'il soit sans sulfate ce tensioactif issu de réactions utilisant l'oxyde d'éthylène qui n'est pas compatible avec une approche écologique et son utilisation en combinaison avec le Sodium coco sulfate ou SLSA entraine la présence de sulfate en grande quantité dans les formulations.

Dans un mode de réalisation, la composition cosmétique solide selon l'invention est caractérisée en ce qu'elle est exempte de sodium cocoyl isethionate ou SCI.

Dans un mode de réalisation, la composition cosmétique solide selon l'invention est caractérisée en ce qu'elle est exempte de Sodium coco sulfate ou SLSA.

L'utilisation des acylglutamates sous forme de sels de sodium dans des formulations solides ne permet pas de solidifier aisément la composition et surtout d'obtenir des formulations qui moussent de façon satisfaisante.

De façon surprenante, une composition solide destinée au nettoyage de la peau ou des phanères comprenant plus de 50% de tensioactifs choisis dans le groupe des acylglutamates sous forme de sels de sodium a pu être mise au point avec des propriétés de solidification et de formation de mousse satisfaisantes.

L'invention concerne une composition solide destinée au nettoyage de la peau ou des phanères comprenant plus de 50% de tensioactifs choisis dans le groupe des acylglutamates sous forme de sels de sodium.

Dans un mode de réalisation, l'invention concerne une composition solide destinée au nettoyage de la peau ou des phanères comprenant de 50% à 60% de tensioactifs choisis dans le groupe des acylglutamates sous forme de sels de sodium.

Dans un mode de réalisation, l'invention concerne une composition solide destinée au nettoyage de la peau ou des phanères dont la teneur totale en acylglutamates est, en pourcentage massique, inférieure à 60 %.

Dans un mode de réalisation, l'invention concerne une composition solide destinée au nettoyage de la peau ou des phanères comprenant plus de 50% de tensioactifs, lesdits tensioactifs comprenant au moins trois acylglutamates sous forme de sels de sodium différents et ladite composition étant exempte de sels de triéthanolamine.

Dans un mode de réalisation, elle concerne une composition, caractérisée en ce que les acylglutamates sont choisis dans les groupe constitué du sodium lauroyl glutamate, du disodium cocoyl glutamate, du sodium myristoyl glutamate et du sodium stearoyl glutamate.

Dans un mode de réalisation, elle concerne une composition, caractérisée en ce que les acylglutamates sont choisis dans les groupe constitué du sodium lauroyl glutamate, du disodium cocoyl glutamate et du sodium myristoyl.

Dans un mode de réalisation, elle concerne une composition, caractérisée en ce que la teneur en sodium lauroyl glutamate est en pourcentage massique comprise entre 25 et 40 %.

Dans un mode de réalisation, elle concerne une composition, caractérisée en ce que la teneur en disodium cocoyl glutamate est en pourcentage massique comprise entre 8 et 25 %.

Dans un mode de réalisation, elle concerne une composition, caractérisée en ce que la teneur en sodium myristoyl glutamate est en pourcentage massique comprise entre 5 et 15 %.

Dans un mode de réalisation, la composition comprend en outre au moins un tensio-actif non ionique choisi dans la famille des glucosides, comme le décylglucoside ou Décyl-β-D-glucopyranoside.

Dans un mode de réalisation, elle concerne une composition, caractérisée en ce que la teneur en décylglucoside ou Décyl-β-D-glucopyranoside est en pourcentage massique comprise entre 5 et 15 %

Dans un mode de réalisation, la composition comprend en outre au moins un tensio-actif conditionneur choisi dans les produits issus de végétaux, comme le mélange désigné sous le code INCI arachidyl / behenyl alcohol/ behenyl betainate esylate.

Dans un mode de réalisation, elle concerne une composition, caractérisée en ce que la teneur en INCI arachidyl / behenyl alcohol/ behenyl betainate esylate est en pourcentage massique comprise entre 3 et 10 %.

Dans un mode de réalisation, l'invention concerne une composition solide destinée au nettoyage de la peau ou des phanères comprenant de 50% à 60% de tensioactifs choisis dans le groupe des acylglutamates sous forme de sels de sodium et comprenant en outre une phase grasse.

Dans un mode de réalisation, ladite phase grasse ou phase huileuse ou phase lipophile ou phase organique, comporte des huiles, des beurres, des cires et des dérivés d'acides gras, d'origine végétale ou minérale, respectivement solides, semi-solides ou liquides à température ambiante.

La teneur en la phase grasse est en pourcentage massique comprise entre 0 et 20%, de préférence entre 3 et 15%.

Il est connu que l'ajout de corps gras altère le pouvoir moussant ; or de façon surprenante, le pouvoir moussant a été conservé sans ajout de savon.

Parmi les beurres végétaux, on citera le beurre de karité, le beurre de cacao, le beurre de Shorea robusta (Beurre de Sal) et parmi les huiles végétales, l'huile d'avocat, de coco, de son de riz, de baie de laurier...

Parmi les cires végétales on citera la cire de carnauba, la cire de riz, la cire de mimosa, la cire de Candellila, la cire d'olive ou de soja.

Parmi les cires on peut également citer la cire d'abeille.

Parmi les dérivés d'acide gras, on citera les esters d'acides gras issus d'huiles végétales comme l'huile de colza ou de navette commercialisés par exemple sous l'INCI Brassica Glycerides ou l'INCI arachidyl / behenyl alcohol/ behenyl betainate esylate utilisés également pour leurs propriétés émolliente et démêlante.

La composition selon l'invention présente également l'avantage d'être aisément hydratable en surface en contact avec de l'eau ou une surface humide au moment de l'utilisation.

Dans un mode de réalisation, la composition cosmétique solide selon l'invention est caractérisée en ce qu'elle comprend en outre une huile essentielle.

Dans un mode de réalisation, la composition cosmétique solide selon l'invention est caractérisée en ce qu'elle comprend entre 0,1 et 1,5 % de ladite huile essentielle, en pourcentage massique de ladite composition cosmétique solide.

Parmi les huiles essentielles utilisées comme parfum mais également pour leurs propriétés sur la peau et les phanères on citera les huiles essentielles de lavande, de neroli, de mandarine, de menthe poivrée, de palmarosa de tea tree et de romarin.

Dans un mode de réalisation, la composition cosmétique solide selon l'invention est caractérisée en ce qu'elle comprend en outre un parfum.

Dans un mode de réalisation, la composition cosmétique solide selon l'invention est caractérisée en ce qu'elle comprend entre 0,1 et 1,5 % dudit parfum, en pourcentage massique de ladite composition cosmétique solide.

Dans un mode de réalisation, la composition cosmétique solide selon l'invention est caractérisée en ce que ladite composition cosmétique solide a une masse comprise entre 10 et 1000 grammes.

Dans un mode de réalisation, la composition cosmétique solide selon l'invention est caractérisée en ce que ladite composition cosmétique solide a une masse comprise entre 50 et 500 grammes.

Dans un mode de réalisation, la composition cosmétique solide selon l'invention est caractérisée en ce que ladite composition cosmétique solide a une masse comprise entre 50 et 250 grammes.

Dans un mode de réalisation, la composition cosmétique solide selon l'invention est caractérisée en ce que ladite composition cosmétique solide a un volume compris entre 7 et 1200 cm3.

Dans un mode de réalisation, la composition cosmétique solide selon l'invention est caractérisée en ce que ladite composition cosmétique solide a un volume compris entre 50 et 600 cm3.

Dans un mode de réalisation, la composition cosmétique solide selon l'invention est caractérisée en ce que ladite composition cosmétique solide a un volume compris entre 100 et 300 cm3.

Dans un mode de réalisation, la composition cosmétique solide selon l'invention est caractérisée en ce qu'elle se présente sous forme d'un parallélépipède, d'un cylindre, d'une barre, d'une sphère ou d'un cube.

Dans un mode de réalisation, la composition cosmétique solide selon l'invention est obtenue par mélange des ingrédients sans étape de dissolution.

Dans un mode de réalisation, la composition cosmétique solide selon l'invention est obtenue par mélange des ingrédients avec une étape de mise en fusion de la phase grasse.

Dans un mode de réalisation, la composition cosmétique solide selon l'invention est obtenue par moulage.

Dans un mode de réalisation, la composition cosmétique solide selon l'invention est obtenue par extrusion suivie d'un découpage.

Dans un mode de réalisation, la composition cosmétique solide selon l'invention est obtenue par extrusion sous forme divisée comme des granules.

Dans un mode de réalaisation la composition selon l'invention est caractérisée en ce qu'elle est obtenue par introduction de l'intégralité des constituants dans une extrudeuse et le mélange est extrudé à une température inférieure à 80°C, de préférence inférieure à 60°C.

Pour obtenir les formes souhaitées on peut fixer au niveau de la sortie de l'extrudeuse, un embout spécifique permettant de donner au boudin issu de l'extrudeuse la forme désirée. Ce boudin, dont la forme est adaptée à la forme finale souhaitée, est par la suite découpé à la taille souhaitée.puis divisé sous forme de parallélépipèdes, de cylindres, de losanges, de barres, de sphères ou de cubes de masse comprise entre 10 et 1000 grammes.

Dans un mode de réalisation, la composition cosmétique solide selon l'invention est une composition cosmétique solide nettoyante utilisée en présence d'eau, par exemple sous une douche, sous un filet d'eau ou un bain.

L'invention concerne également l'utilisation d'une composition cosmétique solide selon l'invention.

L'invention concerne également l'utilisation d'une composition cosmétique solide selon l'invention, pour le lavage du tégument (peau, phanères).

L'invention concerne également l'utilisation d'une composition cosmétique solide selon l'invention, pour le lavage de la peau.

L'invention concerne également l'utilisation d'une composition cosmétique solide selon l'invention, pour le lavage des phanères.

L'invention concerne également l'utilisation d'une composition cosmétique solide selon l'invention, pour le lavage des cheveux.

L'invention concerne également l'utilisation d'une composition cosmétique solide selon l'invention, pour le lavage des muqueuses.

La composition selon l'invention peut en outre comprendre tout additif susceptible d'être utilisé dans le domaine des compositions destinées à la peau ou aux phanères.

En particulier, elle peut comprendre des huiles minérales, animales, végétales, synthétiques ou siliconées, lesdites huiles pouvant être volatiles ou non, des corps gras pâteux, des conditionneurs, des émollients, des cires d'origine animale, minérale, végétale ou synthétique, des gélifiants, des parfums des polymères éventuellement filmogènes, des agents régulateurs de pH, des séquestrants, des pigments, des charges, des actifs cosmétiques ou pharmaceutiques.

Parmi les huiles et les corps gras on citera notamment les huiles d'avocat, de noix de coco ou le beurre de Karité.

Parmi les émollients on citera notamment les esters d'acides aminés comme le L-Proline, 5-oxo-, 2-hydroxy-3-[(1-oxo-9-octadecenyl)oxy]propyl ester, (Z) ou PCA glycéryl oleate, ou les mélanges de mono, di et triglycérides issus de différentes formes d'huile de Brassica commercialisées sous la dénomination INCI brassica glycerides.

La nature de ce ou ces additifs et/ou leur quantité, seront adaptées de façon à ce que les propriétés recherchées de la composition selon l'invention ne soient pas ou substantiellement pas, altérées par l'adjonction envisagée.

### Exemples

Les compositions selon l'invention sont préparées selon le procédé ci-après décrit.

Deux phases sont préparées, une phase A, obtenue par mélange des ingrédients solide et ou légèrement hydratés

Puis une phase B est préparée par mélange et fusion, d'une phase grasse.

Les deux phases sont ensuite intimement mélangées jusqu'à l'obtention d'une pâte homogène.

La pâte est ensuite extrudée à une température inférieure à 80°C choisie en fonction des constituants des exemples ci-après et découpée dans la forme souhaitée losanges pour les shampoings ou cylindres pour les pains lavants. Les produits sont ensuite mis au séchage pendant 24h.

Selon ce procédé les formulations suivantes sont préparées.

### A - Pain lavant douceur

**Tableau 1**

| **Phases** | **Désignation** | **INCI** | **% MP Qsp 100** |
|---|---|---|---|
| A | Sodium myristoyl glutamate poudre | Sodium Myristoyl Glutamate | 8,00 |
| A | Amidon de maïs BIO | Zea Mays Starch | 8,00 |
| A | FRAG Divine Vanille | Parfum | 1,40 |
| A | Mousse de sucre sans palme | Decyl Glucoside, Aqua | 10,00 |
| A | Vitamine E | Helianthus Annuus Seed Oil, Tocopherol | 0,20 |
| A | Argile blanche surfine | Kaolin | 1,00 |
| A | Avoine colloïdale BIO poudre | Avena sativa kernel flour | 1,00 |
| A | Sodium lauroyl glutamate | Sodium Lauroyl Glutamate | 27,00 |
| A | Sodium cocoyl glutamate en poudre | Disodium Cocoyl Glutamate, Aqua | 20,00 |
| A | HV Son de riz BIO | Oryza Sativa Bran Oil | 4,00 |
| A | FRAG Vanille gourmande | Parfum | 1,40 |
| B | Beurre de Cacao blanc pastilles BIO | Theobroma Cacao Seed Butter | 5,00 |
| B | Brassica Glycerides | Brassica Glycerides | 13,00 |

### B - Pain lavant pureté

**Tableau 2**

| **Phases** | **Désignation** | **INCI** | **% MP Qsp 100** |
|---|---|---|---|
| A | Mousse de sucre sans palme | Decyl Glucoside, Aqua | 10,00 |
| A | Neem BIO poudre | Azadirachta Indica Leaf Extract | 4,25 |
| A | Vitamine E | Helianthus Annuus Seed Oil, Tocopherol | 0,20 |
| A | Sodium cocoyl glutamate en poudre | Disodium Cocoyl Glutamate, Aqua | 21,00 |
| A | HV Nigelle BIO | Nigella sativa seed oil | 4,00 |
| A | Sodium myristoyl glutamate poudre | Sodium Myristoyl Glutamate | 8,00 |
| A | HE Menthe verte BIO | Mentha Spicata Herb Oil | 0,20 |
| A | FRAG Thé vert | Parfum | 2,50 |
| A | HE Tea Tree BIO | Melaleuca Alternifolia Leaf Oil | 0,05 |
| A | Argile verte montmorillonite surfine | Montmorillonite, Illite | 4,80 |
| A | Sodium lauroyl glutamate | Sodium Lauroyl Glutamate | 27,00 |
| B | Brassica Glycerides | Brassica Glycerides | 13,00 |
| B | Beurre Sal BIO | Shorea Robusta Seed Butter | 5,00 |

### C - Shampoing nourrissant

**Tableau 3**

| **Phases** | **Désignation** | **INCI** | **% MP Qsp 100** |
|---|---|---|---|
| A | Amidon de maïs BIO | Zea Mays Starch | 3,05 |
| A | FC Theobroma | Parfum | 0,25 |
| A | FRAG Délice de Coco | Parfum | 3,50 |
| A | HV Avocat BIO | Persea Gratissima Oil | 2,00 |
| A | HV Coco BIO | Cocos Nucifera Oil | 2,00 |
| A | Lait de coco en poudre BIO | Cocos nucifera milk powder | 2,00 |
| A | Mousse de sucre sans palme | Decyl Glucoside, Aqua | 10,00 |
| A | PCA Glyceryl oleate | Pca Glyceryl Oleate | 1,50 |
| A | Sodium cocoyl glutamate en poudre | Disodium Cocoyl Glutamate, Aqua | 10,00 |
| A | Sodium lauroyl glutamate | Sodium Lauroyl Glutamate | 37,00 |
| A | Sodium myristoyl glutamate poudre | Sodium Myristoyl Glutamate | 10,00 |
| A | Tego Solve 90 | Polyglyceryl-6 Caprylate, Polyglyceryl-4 Caprate, Aqua | 5,00 |
| A | Vitamine E | Helianthus Annuus Seed Oil, Tocopherol | 0,20 |
| B | Beurre Karité BIO raffiné | Butyrospermum Parkii Butter | 1,00 |
| B | Brassica Glycerides | Brassica Glycerides | 2,50 |
| B | CosmeGreen ES 1822+ | Arachidyl/Behenyl Alcohol, Arachidyl/Behenyl Betainate Esylate | 10,00 |

### D - Shampoing purifiant

**Tableau 4**

| **Phases** | **Désignation** | **INCI** | **% MP Qsp 100** |
|---|---|---|---|
| A | Amidon de maïs BIO | Zea Mays Starch | 4,30 |
| A | Argile verte montmorillonite surfine | Montmorillonite, Illite | 1,70 |
| A | FRAG Thé vert | Parfum | 1,00 |
| A | HE Menthe verte BIO | Mentha Spicata Herb Oil | 0,08 |
| A | HE Pamplemousse blanc BIO | Citrus Paradisi Peel Oil | 0,56 |
| A | HE Romarin à verbénone BIO | Rosmarinus Officinalis Leaf Oil (Verbenone) | 0,16 |
| A | HV Baies de Laurier BIO | Laurus Nobilis Fruit Oil | 1,00 |
| A | Mousse de sucre sans palme | Decyl Glucoside, Aqua | 10,00 |
| A | Ortie piquante BIO poudre | Urtica Dioica Leaf Extract | 4,50 |
| A | PCA Glyceryl oleate | Pca Glyceryl Oleate | 1,50 |
| A | Sodium cocoyl glutamate en poudre | Disodium Cocoyl Glutamate, Aqua | 10,00 |
| A | Sodium lauroyl glutamate | Sodium Lauroyl Glutamate | 37,00 |
| A | Sodium myristoyl glutamate poudre | Sodium Myristoyl Glutamate | 10,00 |
| A | Tego Solve 90 | Polyglyceryl-6 Caprylate, Polyglyceryl-4 Caprate, Aqua | 5,00 |
| A | Vitamine E | Helianthus Annuus Seed Oil, Tocopherol | 0,20 |
| B | Brassica Glycerides | Brassica Glycerides | 7,00 |
| B | CosmeGreen ES 1822+ | Arachidyl/Behenyl Alcohol, Arachidyl/Behenyl Betainate Esylate | 6,00 |

### E - Shampoing fortifiant

**Tableau 5**

| **Phases** | **Désignation** | **INCI** | **% MP Qsp 100** |
|---|---|---|---|
| A | Mousse de sucre sans palme | Decyl Glucoside, Aqua | 10,00 |
| A | Manjishta poudre | Rubia Cordifolia Root Powder | 0,30 |
| A | HV Moutarde BIO | Brassica Nigra Seed (Oil) | 3,00 |
| A | Sodium lauroyl glutamate | Sodium Lauroyl Glutamate | 37,00 |
| A | Sodium cocoyl glutamate en poudre | Disodium Cocoyl Glutamate, Aqua | 10,00 |
| A | Vitamine E | Helianthus Annuus Seed Oil, Tocopherol | 0,10 |
| A | PCA Glyceryl oleate | Pca Glyceryl Oleate | 1,50 |
| A | Amla BIO poudre # | Emblica Officinalis Fruit Powder | 1,00 |
| A | Tego Solve 90 | Polyglyceryl-6 Caprylate, Polyglyceryl-4 Caprate, Aqua | 5,00 |
| A | Amidon de maïs BIO | Zea Mays Starch | 4,10 |
| A | FRAG Mangue tropicale | Parfum | 0,98 |
| A | HE Cannelle tamala BIO | Cinnamomum Tamala Leaf Oil | 0,05 |
| A | HV Hibiscus BIO | Hibiscus Sabdariffa Seed Oil | 2,00 |
| A | Sodium myristoyl glutamate poudre | Sodium Myristoyl Glutamate | 10,00 |
| A | HE Orange douce BIO | Citrus Sinensis Peel Oil Expressed | 1,97 |
| B | CosmeGreen ES 1822+ | Arachidyl/Behenyl Alcohol, Arachidyl/Behenyl Betainate Esylate | 6,00 |
| B | Brassica Glycerides | Brassica Glycerides | 7,00 |

## Revendications

1. Composition solide destinée au nettoyage de la peau ou des phanères comprenant plus de 50% de tensioactifs, lesdits tensioactifs comprenant au moins trois acylglutamates sous forme de sels de sodium différents et ladite composition étant exempte de sels de triéthanolamine.

2. Composition selon la revendication 1, **caractérisée en ce que** lesdits acylglutamates de sodium sont choisis dans le groupe constitué du sodium lauroyl glutamate, du disodium cocoyl glutamate, du sodium myristoyl glutamate et du sodium stearoyl glutamate

3. Composition selon la revendication 1, **caractérisée en ce que** lesdits acylglutamates de sodium sont choisis dans le groupe constitué du sodium lauroyl glutamate, du disodium cocoyl glutamate et du sodium myristoyl glutamate.

4. Composition selon l'une quelconque des revendications précédentes, **caractérisée en ce qu'**elle est exempte de savons.

5. Composition selon l'une quelconque des revendications précédentes, **caractérisée en ce qu'**elle ne comporte pas de support.

6. Composition selon l'une quelconque des revendications précédentes, **caractérisée en ce que** la teneur totale en acylglutamates est est en pourcentage massique inférieure à 60 %.

7. Composition selon l'une quelconque des revendications précédentes, **caractérisée en ce que** la teneur en sodium lauroyl glutamate est en pourcentage massique comprise entre 25 et 40 %.

8. Composition selon l'une quelconque des revendications précédentes, **caractérisée en ce que** la teneur en disodium cocoyl glutamate est en pourcentage massique comprise entre 8 et 25 %.

9. Composition selon l'une quelconque des revendications précédentes, **caractérisée en ce que** la teneur en sodium myristoyl glutamate est en pourcentage massique comprise entre 5 et 15 %.

10. Composition selon l'une quelconque des revendications précédentes, **caractérisée en ce qu'**elle comporte une phase grasse.

11. Composition selon l'une quelconque des revendications précédentes, **caractérisée en ce qu'**elle est obtenue par mélange des ingrédients sans étape de dissolution.

12. Composition selon l'une quelconque des revendications précédentes, **caractérisée en ce qu'**elle est obtenue par mélange des ingrédients avec une étape de mise en fusion de la phase grasse.

13. Composition selon l'une quelconque des revendications précédentes, **caractérisée en ce qu'**elle est obtenue par introduction de l'intégralité des constituants dans une extrudeuse et le mélange est extrudé à une température inférieure à 80°C, de préférence inférieure à 60 °C.
